# FASCICULE DE BREVET EUROPEEN

(11) **EP 3 760 114 B1**
(45) Date de publication et mention de la délivrance du brevet: **24.01.2024**
(21) Numéro de dépôt: 20183499.1
(22) Date de dépôt: 01.07.2020
(51) Int. Cl.: A61B 5/283, A61N 1/39, A61N 1/05, A61B 5/00, A61B 5/30, A61B 5/352, A61B 5/363

(54) **DISPOSITIF MEDICAL IMPLANTABLE SOUS CUTANE POUR TRAITER DES SIGNAUX D'UN DISPOSITIF MEDICAL IMPLANTABLE SOUS CUTANE**
SUBKUTAN IMPLANTIERBARE MEDIZINISCHE VORRICHTUNG ZUR VERARBEITUNG DER SIGNALE EINER SUBKUTAN IMPLANTIERBAREN MEDIZINISCHEN VORRICHTUNG
SUBCUTANEOUS IMPLANTABLE MEDICAL DEVICE FOR PROCESSING SIGNALS FROM A SUBCUTANEOUS IMPLANTABLE MEDICAL DEVICE

(30) Priorité: 05.07.2019 FR 1907534
(43) Date de publication de la demande: 06.01.2021
(73) Titulaire: Sorin CRM SAS, 92140 Clamart (FR)
(72) Inventeur: LEGAY, Thierry, 91640 Fontenay Les Briis (FR); CORDERO ALVAREZ, Rafael, 75014 Paris (FR); FEUERSTEIN, Delphine, 92100 Boulogne Billancourt (FR)
(74) Mandataire: Ungerer, Olaf

(56) Documents cités:
- EP-A1- 2 368 493
- EP-A1- 2 742 971
- US-A1- 2005 049 644
- US-A1- 2019 111 265
- US-A1- 2019 117 990
- US-B2- 8 577 455

## Description

La présente invention se rapporte à un dispositif cardiaque implantable sous cutané, en particulier un défibrillateur implantable sous cutané tel que décrit à la revendication 1.

Les défibrillateurs automatiques implantables classiques, c'est-à-dire de type transveineux, aussi abréviés ICD pour « Implantable Cardioverter-Defibrillator » en anglais, comprennent un générateur d'impulsions de défibrillation et une unité de contrôle à microprocesseur logés dans un boîtier métallique généralement implanté de manière sous-cutanée dans la poche pectorale. Ce boîtier est connecté à une ou plusieurs sondes qui sont introduites dans la veine sous-clavière où elles atteignent finalement le coeur. À l'intérieur du coeur, les extrémités distales des sondes sont fixées aux parois internes des cavités cardiaques, où elles peuvent enregistrer des électrogrammes (EGM) reflétant le fonctionnement électrophysiologique du coeur. Sur la base de ceux-ci, un traitement sous forme de défibrillation (choc de défibrillation) est administré (ou interrompu) afin de mettre fin à une tachyarythmie ventriculaire représentant un danger de mort, telle que la tachycardie ventriculaire et la fibrillation ventriculaire.

Les éléments les plus faibles de ces défibrillateurs automatiques implantables transveineux (ainsi que des stimulateurs cardiaques et dispositifs similaires) sont les sondes intracardiaques. La fracture de sonde est d'ailleurs l'une des causes les plus courantes de dysfonctionnement du stimulateur cardiaque. L'extraction d'une sonde ICD (ou d'un stimulateur cardiaque) implantée est une procédure à forte morbidité et mortalité élevée, et est donc généralement uniquement effectuée en cas d'infection systémique grave ne pouvant pas être traitée avec des antibiotiques. Dans la majorité des cas, les sondes fracturées seront déconnectées du dispositif et laissées dans le coeur. Une nouvelle sonde est ensuite implantée à côté de l'ancienne et connectée au défibrillateur automatique implantable. Cependant, cette solution n'est possible que s'il reste encore suffisamment d'espace dans la veine, car la présence de davantage de sondes peut entraîner une occlusion veineuse. Par conséquent, l'utilisation de sondes intracardiaques n'est pas idéalement adaptée à de jeunes patients, qui pourraient avoir besoin d'une multitude de sondes au cours de leur vie.

Une solution aux problèmes associés aux sondes intracardiaques, énumérés ci-dessus, consiste à les remplacer par des sondes sous-cutanées. Ainsi, en l'absence de contact avec le coeur ou le sang, le risque d'infection systémique est éliminé et les veines ne sont plus obstruées. En outre, contrairement aux sondes intracardiaques, l'extraction des sondes sous-cutanées est moins traumatique et ne comporte pas de risque de mortalité, car les sondes sous-cutanées ne touchent pas le coeur. De ce fait, les sondes peuvent être retirées en toute sécurité en cas de fracture et remplacées par de nouvelles sondes sous-cutanées, sans risque pour le patient.

Les principaux défis des dispositifs implantables sous-cutanés sont liés à la réduction du rapport signal sur bruit des signaux enregistrés par voie sous-cutanée et à l'augmentation de l'énergie requise pour une défibrillation réussie. Les dispositifs implantables sous-cutanés enregistrent des électrocardiogrammes (ECG) sous-cutanés - plutôt que des EGM - qui capturent une activité électrophysiologique spatialement moyennée en champ lointain. Les ondes P et les ondes T sont alors plus importantes dans les signaux sous-cutanés par rapport aux ondes R, rendant plus difficile la détection de l'intervalle RR sur laquelle de nombreux algorithmes de détection de tachyarythmie sont basés. Les sources de bruit non cardiaques, telles que les myopotentiels, peuvent également altérer les signaux sous-cutanés et interférer avec les algorithmes de détection, ce qui perturbe le traitement. De plus, les signaux sous-cutanés ont tendance à être plus sensibles aux changements de posture que les signaux intracardiaques. Dans l'ensemble, ces difficultés se traduisent par une procédure de détection plus complexe que pour les défibrillateurs automatiques implantables classiques. Des algorithmes ou des procédés de traitement de signaux d'électrogramme endocavitaires, tels que connus de EP 2 105 843 A1 et de EP 2 368 493 A1, sont configurés pour permettre de discriminer une véritable tachycardie ventriculaire (TV) d'une tachycardie supraventriculaire (TSV). Cependant, ces algorithmes connus ne sont pas adaptés à la détection de tachyarythmie à partir de signaux recueillis par voie sous cutanée. En effet, parce ce que les défibrillateurs sous-cutanés sont davantage susceptibles d'être soumis à la surdétection de bruit et des ondes P, T ; il est nécessaire, dans le cas particulier des défibrillateurs sous-cutanés, de minimiser le risque de diagnostiquer une TV ou une fibrillation ventriculaire (FV) lorsqu'il y a surdétection de bruit ou des ondes P, T. Une interprétation erronée des signaux recueillis pourrait entraîner, par exemple dans un défibrillateur automatique implantable, un choc inapproprié, pouvant être traumatique, voire délétère pour le patient.

US 8,577,455 B2 divulgue un dispositif pour la détection d'arythmies chez un patient, incluant des électrodes sous-cutanées et un processeur capable de traiter les signaux cardiaques. US 2005/049644 A1 divulgue un système de traitement cardiaque implantable, capable de sélectionner le vecteur électrode le plus approprié pour la détection en continu et améliorer la détection d'évènements cardiaques.

Du fait des limitations susmentionnées, il y a un besoin d'améliorer le traitement de signaux recueillis via une sonde sous cutanée, en particulier pour pouvoir confirmer la présence ou non d'une tachyarythmie et différencier une surdétection d'ondes T ou P (ou de bruit) d'une tachyarythmie.

L'objet de la présente invention est donc d'améliorer le traitement des signaux recueillis au moyen d'une sonde sous-cutanée d'un dispositif médical sous cutané, en particulier d'améliorer la sensibilité et la spécificité de la détection et de la discrimination des épisodes de tachyarythmie enregistrés à partir d'une sonde sous-cutanée.

L'objet de la présente invention est atteint par un dispositif médical actif implantable sous cutané, en particulier un défibrillateur cardiaque sous cutané, comprenant : un boîtier, une sonde implantable sous cutanée connectée au boîtier, la sonde implantable sous cutanée comprenant une pluralité d'électrodes de détection formant au moins deux dipôles à partir desquels au moins deux signaux électriques sont concurremment recueillis ; le premier dipôle ayant une longueur inférieure à celle du deuxième dipôle ; le dispositif comprenant en outre un contrôleur configuré pour déterminer la présence ou non d'une tachyarythmie en déterminant un critère de similarité à partir des signaux électriques recueillis concurremment via le premier dipôle et via le deuxième dipôle pendant une série définie de cycles cardiaques, telle que la détection d'un pic de dépolarisation, correspondant à la détection d'une onde R, est réalisée via le premier dipôle.

La détection d'un pic de dépolarisation à partir des signaux recueillis via le premier dipôle permet d'améliorer la qualité de la détection de l'onde R par voie sous-cutanée car le premier dipôle est le plus court des deux dipôles de la sonde sous-cutanée, et, est donc moins exposé au risque de surdétection de bruit ou d'erreur lors de la détection du complexe QRS par rapport au deuxième dipôle. En effet, la distance couverte entre les électrodes du premier dipôle est réduite, ce qui diminue les risques d'altération des signaux par une source externe. Il y a, par exemple, moins de masse musculaire entre les électrodes pouvant introduire des myopotentiels.

La présente invention, relative à un dispositif médical actif implantable sous cutané, peut être davantage améliorée grâce aux modes de réalisation suivants.

Selon un mode de réalisation de l'invention, les signaux recueillis via le premier dipôle et le deuxième dipôle de la sonde implantable sous cutanée peuvent être considérés sur une fenêtre temporelle comprenant un complexe QRS et centrée sur la détection de l'onde R, la détection de l'onde R étant uniquement réalisée via le premier dipôle.

Ainsi, l'intervalle R-R des signaux recueillis via le premier dipôle peut être déterminé et une fenêtre temporelle, par exemple de 80 à 150 millisecondes, en particulier de 100 millisecondes, peut être centrée sur la détection de l'onde R réalisée via le premier dipôle. La détection de l'onde R n'est ainsi pas réalisée sur le deuxième dipôle de manière à ne pas ajouter davantage de coût de calcul. Les signaux du deuxième dipôle recueillis concurremment à ceux du premier dipôle, en particulier uniquement pendant une fenêtre temporelle, peuvent être enregistrés dans la mémoire du dispositif.

Selon l'invention, le contrôleur est configuré pour combiner les signaux recueillis via le premier dipôle et les signaux recueillis via le deuxième dipôle, et à partir de la représentation des signaux recueillis via le deuxième dipôle en fonction des signaux recueillis via le premier dipôle, de déterminer une courbe bidimensionnelle paramétrée en fonction du temps représentative de l'activité cardiaque d'un patient, le critère de similarité étant défini par la comparaison, en particulier une corrélation, entre ladite courbe bidimensionnelle et une courbe bidimensionnelle de référence représentative en rythme sinusal normal.

La détermination de la courbe bidimensionnelle par le contrôleur permet de combiner des signaux recueillis selon le premier dipôle avec ceux recueillis selon le deuxième dipôle : il est ainsi rendu possible de prendre en compte simultanément les informations venant des deux dipôles de la sonde sous-cutanée, notamment en considérant - selon le dipôle - différentes morphologies de signaux électriques, ce qui contribue à l'obtention de paramètres davantage pertinents relatifs à l'origine du signal recueilli. Ces paramètres relatifs à l'origine du signal recueilli participent notamment à la détermination du critère de similarité.

Selon un mode de réalisation de l'invention, le contrôleur peut en outre être configuré pour identifier la présence ou non de bruit indésirable dans les signaux recueillis en fonction du changement de signe d'au moins une des coordonnées d'un vecteur tangent entre chaque point successif d'une pluralité de points de ladite courbe bidimensionnelle. Plus précisément, le contrôleur peut être configuré pour identifier la présence de bruit indésirable en déterminant le nombre de fois qu'au moins une des coordonnées du vecteur tangent change de signe entre chaque point successif de la pluralité de points de ladite courbe bidimensionnelle et en comparant ce nombre à un nombre seuil prédéterminé indicateur de bruit indésirable.

En effet, dans le cas d'un cycle cardiaque non bruité, il existe entre les signaux détectés sur les deux dipôles un rapport qui fait que la courbe bidimensionnelle soit représentée de façon essentiellement régulière. À l'inverse, dans le cas d'un cycle cardiaque bruité, c'est-à-dire comprenant des artefacts d'origine extracardiaque, la courbe bidimensionnelle peut être représentée de façon erratique. Une telle représentation erratique peut être caractérisée par le nombre de fois qu'au moins une des coordonnées du vecteur tangent change de signe entre chaque point successif de la courbe bidimensionnelle. Le contrôleur est ainsi adapté pour identifier la présence de bruit dans les signaux recueillis au moyen d'un calcul rapide et rentable en termes de ressources logicielles. Le contrôleur du dispositif pourrait être configuré pour passer dans un mode dit « mode bruit » pendant un certain lapse de temps, par exemple pendant 30 secondes, en augmentant le seuil de détection des ondes R ou en allongeant les périodes réfractaires.

Selon un mode de réalisation de l'invention, le contrôleur peut en outre être configuré pour déterminer la présence ou non d'une tachyarythmie en déterminant un critère de majorité qui est déterminé à partir du critère de similarité en calculant le nombre de cycles semblables entre les signaux recueillis et des signaux de référence représentatifs d'un cycle sinusal normal.

Ainsi, selon le critère de majorité, la confirmation de la présence d'un épisode de tachyarythmie peut être déduite. Inversement, il peut être conclu à une surdétection de l'onde T ou/et de l'onde P, ce qui ne requiert pas de traitement particulier. Le dispositif est ainsi configuré pour pouvoir différencier une surdétection d'ondes T ou P (ou de bruit) d'une tachyarythmie ; ce qui est nécessaire dans le cas particulier des défibrillateurs sous-cutanés qui sont davantage susceptibles à la surdétection de bruit et/ou des ondes P, T que les défibrillateurs transveineux.

Selon un mode de réalisation de l'invention, la sonde implantable sous cutanée peut en outre comprendre une électrode de défibrillation. De plus, le contrôleur peut être configuré pour déclencher une opération de défibrillation au moyen de l'électrode de défibrillation lorsque le critère de similarité indique la présence d'une tachyarythmie à traiter.

Ainsi, le dispositif médical implantable sous cutanée est adapté non seulement pour détecter un épisode de tachyarythmie mais également pour traiter une telle condition, en délivrant si nécessaire des impulsions électriques de défibrillation.

Selon un mode de réalisation de l'invention, le dispositif médical actif implantable sous cutané comprend en outre un accéléromètre ou/et un gyroscope de manière à ce que le contrôleur est configuré pour déterminer la position du patient au moyen de l'accéléromètre ou/et du gyroscope ;
le contrôleur étant en outre configuré pour déterminer le critère de similarité en comparant ladite courbe bidimensionnelle à une courbe bidimensionnelle de référence dans ladite position déterminée et représentative en rythme sinusal normal.

Les signaux sous-cutanés sont plus sensibles à la posture que les signaux endocavitaires. Ainsi, en permettant la détection de la position du patient, l'interprétation des signaux électriques recueillis peut être davantage affinée, et donc améliorée.

Est aussi divulgué ici, un procédé, mis en oeuvre par le controleur, pour traiter des signaux électriques recueillis concurremment sur un premier dipôle et un deuxième dipôle formés par des électrodes d'une sonde implantable sous cutanée d'un dispositif médical implantable sous cutané dans le domaine temporel pendant une série définie de cycles cardiaques, comprenant les étapes de : 1) déterminer une courbe bidimensionnelle paramétrée en fonction du temps représentative de l'activité cardiaque d'un patient en traçant les signaux recueillis via le deuxième dipôle en fonction des signaux recueillis via le premier dipôle et déterminer un vecteur tangent en une pluralité de point de ladite courbe bidimensionnelle ; et 2) identifier la présence ou non de bruit indésirable dans les signaux recueillis en fonction du changement de signe d'au moins une des coordonnées du vecteur tangent entre chaque point successif de la pluralité de points de ladite courbe bidimensionnelle ; et/ou 3) confirmer la présence ou non d'un épisode de tachyarythmie selon un critère de similarité entre ladite courbe bidimensionnelle et une courbe bidimensionnelle de référence représentative d'un rythme sinusal normal.

L'étape 1) de détermination de la courbe bidimensionnelle permet de combiner des signaux recueillis selon un premier dipôle avec ceux recueillis selon un deuxième dipôle : il est ainsi rendu possible de prendre en compte simultanément les informations venant de deux dipôles d'une sonde sous-cutanée, notamment en considérant - selon le dipôle - différentes morphologies de signaux électriques, ce qui contribue à l'obtention de paramètres davantage pertinents relatifs à l'origine du signal recueilli. De plus, les signaux recueillis par voie sous-cutanée étant particulièrement exposés à des bruits indésirables, l'étape 2) du procédé permet d'identifier la présence ou non de bruit indésirable, et ce, au moyen de cette même courbe bidimensionnelle, ce qui permet de réduire les coûts de calcul en termes de ressources logicielles, parce que les cycles bruités ne sont pas traités ou pris en considération lors du traitement des signaux recueillis. Enfin, l'étape 3) du procédé permet d'effectuer la discrimination entre présence ou non d'une tachyarythmie, cette discrimination étant opérée elle-aussi à partir de ladite courbe bidimensionnelle. Le procédé de traitement de signaux de la présente invention est ainsi spécifiquement adapté et optimisé pour un dispositif médical implantable sous-cutané.

Le procédé, mis en oeuvre par le controleur, pour traiter des signaux électriques, peut être davantage amélioré grâce aux modes de réalisation suivants.

Selon un mode de réalisation, l'étape 1) du procédé peut être précédée d'une étape initiale d'analyse, en particulier une analyse du rythme cardiaque et/ou de la fréquence cardiaque, afin de détecter la présence potentielle d'un épisode de tachyarythmie dans les signaux recueillis, tel qu'à l'étape initiale d'analyse les signaux recueillis via l'un des dipôles de la sonde implantable sous cutanée sont comparés à un seuil prédéterminé de tachyarythmie.

En particulier, cette étape initiale de comparaison est mise en oeuvre pour les signaux du dipôle qui comprend la détection du complexe QRS, en particulier la détection de l'onde R. Cette étape initiale de comparaison comprend ainsi la comparaison de l'intervalle R-R avec le seuil prédéterminé de tachyarythmie. Cette étape ne nécessitant que la détection de l'onde R sur un des deux dipôles, les coûts de calcul du procédé selon la présente invention peuvent être minimisés.

Selon un mode de réalisation, l'étape 1), l'étape 2) et/ou l'étape 3) peuvent être réalisées seulement si la présence potentielle d'un épisode de tachyarythmie dans les signaux recueillis est détectée à l'étape initiale d'analyse.

Ainsi, les étapes 1), 2) et 3) du procédé pour traiter des signaux ne sont pas réalisées systématiquement mais que si la présence d'un épisode de tachyarythmie est suspectée. Cela permet de réduire davantage les coûts de calcul du procédé, en évitant des étapes de calculs inutiles.

Selon un mode de réalisation, à l'étape 2) la présence de bruit indésirable peut être identifiée si le nombre de fois qu'au moins une des coordonnées du vecteur tangent change de signe entre chaque point successif de la pluralité de points de ladite courbe bidimensionnelle est supérieur à un nombre seuil prédéterminé indicateur de bruit indésirable. En effet, dans le cas d'un cycle cardiaque non bruité, il existe entre les signaux détectés sur les deux dipôles un rapport qui fait que la courbe bidimensionnelle soit représentée de façon essentiellement régulière. À l'inverse, dans le cas d'un cycle cardiaque bruité, c'est-à-dire comprenant des artefacts d'origine extracardiaque, la courbe bidimensionnelle peut être représentée de façon erratique. Une telle représentation erratique peut être caractérisée par le nombre de fois qu'au moins une des coordonnées du vecteur tangent change de signe entre chaque point successif de la courbe bidimensionnelle. L'étape 2) du procédé fournit ainsi une étape de calcul rapide et rentable pour l'identification de bruit indésirable dans les signaux.

Selon un mode de réalisation, le nombre seuil prédéterminé indicateur de bruit indésirable peut être défini relativement à la totalité de la pluralité de points de ladite courbe bidimensionnelle.

Ainsi, l'identification de bruit indésirable peut être déterminée de manière exhaustive en considérant tous les points de la pluralité de points de la courbe bidimensionnelle, c'est-à-dire en prenant en compte l'information contenue à chaque point de la courbe.

Selon un mode de réalisation, l'étape 3) peut être réalisée à partir de signaux dans lesquels la présence potentielle d'un épisode de tachyarythmie a été détectée à l'étape initiale d'analyse ; et dans lesquels du bruit indésirable n'a pas été identifié à l'étape 2.

Afin de pallier aux limitations spécifiquement liées aux signaux recueillis par voie sous-cutanée, comme la surdétection de bruit, le procédé garantit que l'étape pour confirmer la présence ou non d'un épisode de tachyarythmie ne soit enclenchée que pour des signaux d'un cycle considéré valide à l'étape 2), c'est-à-dire pour des signaux d'un cycle non-bruité.

Selon un mode de réalisation, l'identification de la présence de bruit indésirable à l'étape 2) peut être suivie d'une étape de traitement des signaux durant laquelle les cycles cardiaques identifiés comme bruité à l'étape 2) sont ignorés; puis à nouveau de l'étape initiale d'analyse.

Ainsi, la présence de bruits indésirables dans les signaux recueillis ne rend pas nécessairement les signaux inutilisables.

Selon un mode de réalisation, l'étape 3) peut comprendre en outre la détermination d'un critère de majorité qui est déterminé à partir du critère de similarité en déterminant le nombre de cycles semblables entre les signaux recueillis et des signaux de référence représentatifs d'un cycle sinusal normal. De plus, selon un mode de réalisation, le critère de majorité peut être comparé à un seuil de majorité prédéterminé et représentatif de la présence d'un épisode de tachyarythmie permettant de confirmer la présence ou non d'un épisode de tachyarythmie.

Ainsi, selon le critère de majorité et la valeur du seuil de majorité défini, la confirmation de la présence d'un épisode de tachyarythmie peut être déduite. Inversement, il peut être conclu à une surdétection de l'onde T ou/et de l'onde P, ce qui ne requiert pas de traitement particulier. Le procédé de traitement des signaux permet ainsi de différencier une surdétection d'ondes T ou P (ou de bruit) d'une tachyarythmie ; ce qui est nécessaire dans le cas particulier des défibrillateurs sous-cutanés qui sont davantage susceptibles à la surdétection de bruit et/ou des ondes P, T que les défibrillateurs transveineux. L'invention et ses avantages seront expliqués plus en détail dans la suite au moyen de modes de réalisation préférés et en s'appuyant notamment sur les figures d'accompagnement suivantes, dans laquelle :
Figure 1 représente une vue schématisée d'un dispositif implantable sous cutané selon la présente invention ;
Figure 2 représente une vue schématisée et par transparence du dispositif implanté sous cutané selon la présente invention implanté chez un patient;
Figure 3 représente un organigramme du procédé pour traiter des signaux
Figure 4a représente un organigramme détaillé du procédé pour traiter des signaux qui est représenté à la Figure 3 ;
Figure 4b représente un organigramme détaillé du procédé pour traiter des signaux qui est représenté à la Figure 3 ;
Figure 5 représente une courbe bidimensionnelle d'un cycle non-bruité;
Figure 6 représente une courbe bidimensionnelle d'un cycle bruité ;
Figure 7 représente une distribution définie pour discriminer la présence de tachyarythmie.
Figure 8 représente un signal classifié comme rythme sinusal normal selon le procédé de la présente invention;
Figure 9 représente un signal classifié comme un événement de tachycardie selon le procédé;

L'invention va maintenant être décrite plus en détail en utilisant des modes de réalisation avantageux d'une manière exemplaire et en référence aux figures. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

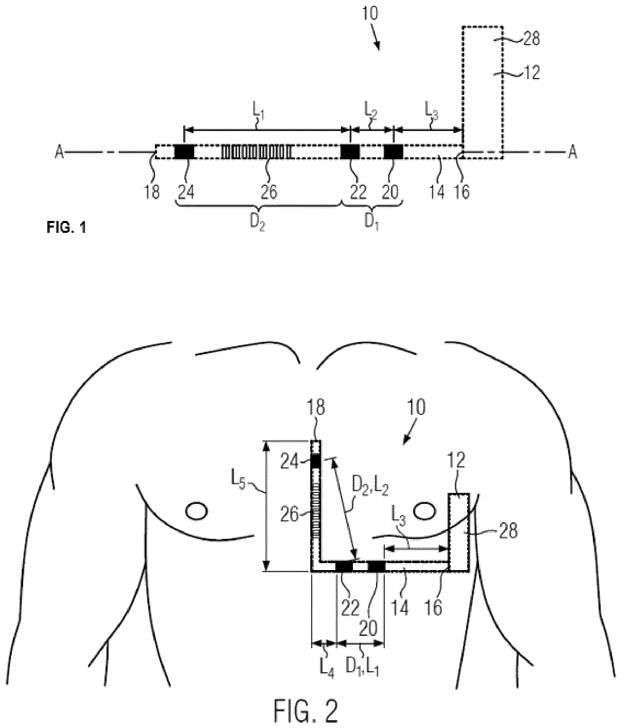

La Figure 1 illustre un dispositif médical implantable sous cutané 10, de type défibrillateur sous cutané 10. La Figure 2 illustre ledit dispositif médical implantable sous cutané 10 dans un état implanté.

Le défibrillateur sous cutané 10 comprend un boîtier 12, générateur d'impulsions, auquel est relié une sonde implantable sous cutanée 14.

La sonde implantable sous cutanée 14 est au moins partiellement souple et comprend deux extrémités 16, 18 : une extrémité proximale 16 qui est connectée au boîtier 12 et une extrémité distale libre 18.

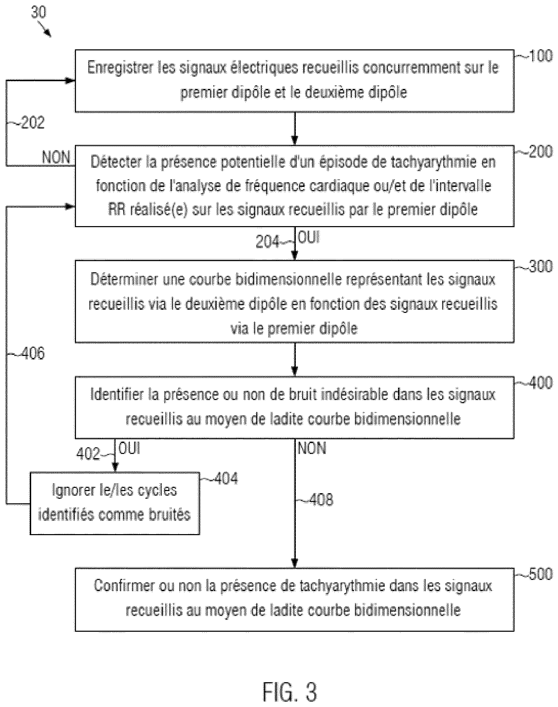

Dans le mode de réalisation illustrée aux Figures 2 et 3, la sonde implantable sous-cutanée 14 comprend trois électrodes de détection 20, 22, 24 et une électrode de défibrillation 26. Dans une variante, la sonde implantable sous-cutanée 14 pourrait comprendre plus que trois électrodes de détection.

La sonde implantable sous cutanée 14 comprend également des fils conducteurs (qui ne sont pas visibles) permettant de connecter électriquement les électrodes 20, 22, 24 de la sonde 14 à des contacts électriques (non visibles) au niveau du boîtier 28 - connu en soi de l'état actuel de la technique.

Les électrodes de détection 20, 22, 24 de la sonde implantable sous-cutanée 14 permettent la détection de signaux électriques utilisés pour déduire l'activité cardiaque d'un patient.

La détection d'activité électrophysiologique par voie sous cutanée est cependant altérée par de nombreux artefacts comme des bruits électriques musculaires ou des interférences avec le milieu extérieur. De plus, la sonde 14 étant de type sous cutanée, les électrodes de détection 20, 22, 24 ne sont pas en contact direct avec le myocarde. Un positionnement adéquat des électrodes de détection 20, 22, 24 permet d'améliorer la qualité des signaux électrophysiologiques détectés et recueillis ; ce qui, en conséquence, permet d'améliorer la qualité de la détection de l'onde R (c'est-à-dire le pic de dépolarisation). De plus, un traitement optimal des signaux recueillis permet d'améliorer davantage la qualité de la détection de l'onde R.

Afin d'améliorer la détection de l'onde R et de minimiser celle des ondes cardiaques P et T, notamment pour faciliter la mesure de l'intervalle R-R, le dispositif implantable sous-cutané 10 comprend un positionnement spécifique des électrodes de détection 20, 22, 24. Précisément, tel qu'illustré aux Figures 2 et 3, une première électrode de détection 20 et une deuxième électrode de détection 22 sont positionnées entre le boîtier 12 et l'électrode de défibrillation 26 ; tandis qu'une troisième électrode de détection 24 est placée entre l'extrémité distale 18 de la sonde 14 et l'électrode de défibrillation 26. L'électrode de défibrillation 26 est ainsi positionnée entre la deuxième électrode de détection 22 et la troisième électrode de détection 24. Ainsi, dans un sens allant de l'extrémité proximale 16 de la sonde 14 à l'extrémité distale 18 de la sonde 14, la sonde comprend dans cet ordre : la première électrode de détection 20, la deuxième électrode de détection 22, l'électrode de défibrillation 26 puis la troisième électrode de détection 24.

Le positionnement spécifique des électrodes de détection 20, 22, 24 va être décrit en terme de longueur en référence à la Figure 1 seulement, représentant la sonde 14 dans un état non-implanté, non-courbé et aligné selon un axe A.

La première électrode de détection 20 et la deuxième électrode de détection 22 forment un premier dipôle D1 de longueur L1.

Selon le mode de réalisation illustrée par les Figures 1 et 2, la deuxième électrode de détection 22 et la troisième électrode de détection 24 forment un deuxième dipôle D2 de longueur L2. Dans une variante, le deuxième dipôle D2 peut être formé par la troisième électrode de détection 24 et la première électrode de détection 20. Dans une autre variante, le boîtier 12 peut servir d'électrode et formé un dipôle avec l'une des électrodes de détection 20, 22, 24.

La longueur L1 du premier dipôle D1 est plus courte que la longueur L2 du deuxième dipôle D2. En particulier, la longueur L1 est compris entre 5 et 50 mm, plus en particulier entre 10 et 20 millimètres, tandis que la longueur L2 est comprise entre 80 et 400 millimètres. De plus, la distance L3 entre la première électrode de détection 20 et le boîtier 12 est comprise entre 80 et 300millimètres.

Le dispositif médical implantable sous-cutané 10 comprend en outre un contrôleur 28 logé dans le boîtier 12. Le contrôleur 28 du dispositif 10 est configuré pour détecter des signaux électrophysiologiques enregistrés simultanément via le premier dipôle D1 et le deuxième dipôle D2 de la sonde implantable sous-cutanée 14. Le contrôleur 28 est configuré pour détecter l'onde R d'un signal cardiaque au niveau du premier dipôle D1.

Le premier dipôle D1 étant plus court que le deuxième dipôle D2, le premier dipôle D1 est moins exposé au risque de surdétection, notamment car il est moins sujet à enregistrer des bruits d'origine musculaire. De plus, le premier dipôle D1 est positionné lors de l'implantation sous-cutanée du dispositif 10 proche et au-dessus de l'encoche cardiaque du poumon gauche, appelée « left lung cardiac notch » en anglais. Cette position particulière du premier dipôle D1 permet de détecter un signal électrophysiologique avec une onde R qui soit davantage distinctif par rapport aux ondes P et T ; les ondes P et T détectées à cet endroit étant minimisées par rapport à l'onde R.

Dans un autre mode de réalisation, le dispositif médical actif implantable sous cutané 10 peut comprendre un accéléromètre ou/et un gyroscope de manière à ce que le contrôleur 28 soit configuré pour déterminer la position du patient au moyen de l'accéléromètre ou/et du gyroscope.

Dans un autre mode de réalisation, la détection de l'onde R via le premier dipôle pourrait être combinée à la détection de signaux cardiaques sur une pluralité de « deuxièmes dipôles », c'est-à-dire sur une pluralité de dipôles qui soient plus longs que le premier dipôle, par exemple : un dipôle formé par la deuxième électrode de détection 22 et la troisième électrode de détection 24, un dipôle formé par la première électrode de détection 20 et la troisième électrode de détection 24 et un dipôle formé entre le boîtier 12 et l'une des électrodes de détection 20, 22, 24.

Les signaux électriques recueillis via le premier dipôle D1 et via le deuxième dipôle D2 servent de signaux d'entrée à un algorithme pour procéder au traitement des signaux dans le but de détecter la présence d'une tachyarythmie. Ces signaux peuvent préalablement faire l'objet d'un prétraitement approprié de filtrage, normalisation et/ou centrage, selon des techniques connues.

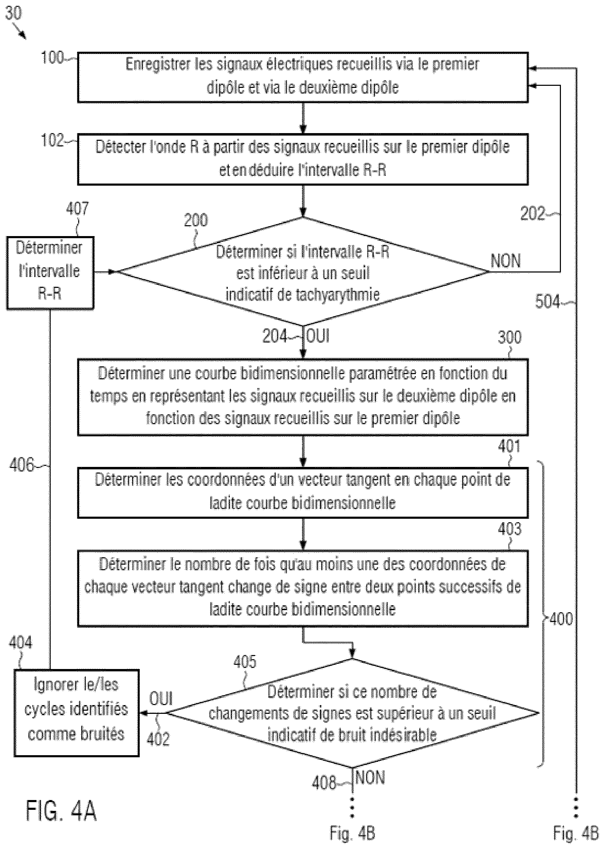

Ce procédé de traitement de signaux est décrit dans ce qui suit de manière générale en référence à la Figure 3, puis davantage en détail en référence aux Figures 4a-b.

Le procédé de traitement de signaux 30 peut être appliqué au dispositif médical implantable sous cutané 10 décrit précédemment en référence aux Figures 1 et 2.

Le procédé 30 illustré à la Figure 3 comprend une première étape 100 au cours de laquelle les signaux concurremment recueillis dans un domaine temporel via le premier dipôle D1 et le deuxième dipôle D2 sont enregistrés. Lesdits signaux peuvent être enregistrés sur une fenêtre de temps prédéterminée et centrée sur l'onde R détectée. Dans une variante, les signaux peuvent être enregistrés de manière continue, notamment en période de suspicion de tachyarythmie. Le contrôleur 28 du dispositif 10 peut lui-même comprendre une mémoire dans laquelle sont stockées lesdits signaux recueillis.

Au cours d'une deuxième étape 200 du procédé 30, la présence potentielle d'un épisode de tachyarythmie est détectée en fonction seulement des signaux recueillis via le premier dipôle D1. Cette deuxième étape 200 peut être basée sur une analyse du rythme cardiaque ou de la fréquence cardiaque. Dans une variante, l'étape 200 peut comprendre une analyse morphologique. Si la présence d'un épisode de tachyarythmie n'est pas suspectée (voir la flèche 202 sur la Figure 3), le procédé 30 retourne à la première étape 100. A l'inverse, si la présence d'un épisode de tachyarythmie est suspectée (voir la flèche 204 sur la Figure 3), le procédé de traitement des signaux 30 se poursuit à une troisième étape 300. Ainsi, les étapes suivantes du procédé pour traiter des signaux 30 ne sont pas inutilement réalisées si la présence d'un épisode de tachyarythmie n'est même pas suspectée. Cela permet notamment de réduire les coûts en termes de puissance et ressources logicielles.

A la troisième étape 300, une courbe bidimensionnelle paramétrée en fonction du temps représentative de l'activité cardiaque d'un patient est déterminée en traçant les signaux recueillis via le deuxième dipôle en fonction des signaux recueillis via le premier dipôle. De plus, un vecteur tangent en une pluralité de point de ladite courbe bidimensionnelle est déterminé. Ledit vecteur peut être un vecteur normalisé.

La troisième étape 300 est suivie d'une quatrième étape 400 au cours de laquelle la présence ou non de bruit indésirable dans les signaux recueillis est identifiées en fonction du changement de signe d'au moins une des coordonnées du vecteur tangent entre chaque point successif de la pluralité de points de ladite courbe bidimensionnelle. Ce procédé de détection de la présence de bruit indésirable permet de réduire les coûts de calcul d'une part par son faible nombre d'étapes et du fait qu'il ne requiert pas une analyse de l'amplitude des signaux recueillis.

Si la présence de bruit indésirable est détectée (voir la flèche 402 sur la Figure 3), le quatrième étape 400 est suivie d'une étape 404 à laquelle la/les composante(s) de bruit indésirable dans les signaux recueillis détecté à l'étape 400 est/sont ignorée(s). L'étape 404 est ensuite suivie de la deuxième étape 200 (voir la flèche 406 sur la Figure 3), telle que décrite précédemment. A l'inverse, si la présence de bruit indésirable dans les signaux n'est pas détectée (voir la flèche 408 sur la Figure 3), le procédé de traitement des signaux 30 se poursuit à une cinquième étape 500.

A la cinquième étape 500, la présence ou non d'un épisode de tachyarythmie est confirmée à partir d'un critère de majorité déterminé selon un critère de similarité entre ladite courbe bidimensionnelle et une courbe bidimensionnelle de référence représentative d'un cycle sinusal normal, et davantage décrit dans ce qui suit.

Le procédé de traitement signal 30 et ses étapes 100 à 500 vont être davantage décrits en référence à l'organigramme des Figures 4a-b. Les numéros de référence ayant le même chiffre de centaine entre eux se réfèrent à une même étape, en particulier à une des étapes 100 à 500 décrites par rapport à la Figure 3 et auxquelles référence est faite.

La première étape 100, à laquelle les signaux concurremment recueillis dans un domaine temporel via le premier dipôle D1 et le deuxième dipôle D2 sont enregistrés, est suivie par une étape 102, au cours de laquelle, après chaque détection d'un pic de dépolarisation, c'est-à-dire après chaque détection d'une onde R, un battement correspondant est isolé par une fenêtre temporelle de largeur fixe et centrée sur ce pic de dépolarisation. La fenêtre temporelle peut par exemple avoir une largeur de 80 à 150 millisecondes centrée sur la détection du pic de l'onde R, en particulier de 100 millisecondes, correspondant à 100 points pour une fréquence d'échantillonnage de 1000 Hz. Cette valeur de 100 millisecondes permet de bien isoler le complexe QRS pour en analyser la morphologie. Le contrôleur 28 du dispositif 10 est configuré pour garder en mémoire une pluralité de cycles détectés successifs. La détection de l'onde R permet notamment de déterminer l'intervalle R-R.

Selon un mode de réalisation avantageux de la présente invention, la détection de l'onde R est uniquement réalisée à partir des signaux recueillis via le premier dipôle D1. La détection de l'onde R n'est ainsi pas réalisée sur le deuxième dipôle D2 de manière à ne pas ajouter de coût de calcul. De plus, le premier dipôle D1 étant plus court que le deuxième dipôle D2, le premier dipôle D1 est moins d'être altéré, notamment car il est moins sujet à enregistrer des bruits d'origine musculaire. En effet, la géométrie du premier dipôle D1 de longueur L1=5 à 50mm, en particulier L1=10 à 20mm, permet de rendre le premier dipôle D1 moins sujet aux myopotentiels. De plus, la position du dipôle D1, lorsque la sonde est implantée, permet d'optimiser la détection du ratio des ondes R/P et R/T et ainsi de réduire le risque de surdétection. Les signaux du deuxième dipôle D2 recueillis concurremment à ceux du premier dipôle D1 sont toutefois gardés en mémoire et utilisés à l'étape 300 du procédé de traitement de signaux 30.

Au cours de la deuxième étape 200 du procédé 30, la présence potentielle d'un épisode de tachyarythmie, basée seulement sur les intervalles R-R des signaux recueillis via le dipôle le plus court D1, est déterminée : si l'intervalle R-R, calculé à l'étape précédente 102, est inférieur à un seuil prédéterminé de tachyarythmie, la présence d'un épisode de tachyarythmie est suspectée. Dans une variante, la présence potentielle d'un épisode de tachyarythmie peut être établie en considérant une moyenne glissante sur une pluralité de cycles, par exemple sur 5 à 20 cycles, en particulier sur 8 cycles. Le seuil prédéterminé de tachyarythmie peut être une valeur fixe ou une valeur ajustable, en particulier programmable pour chaque patient.

Tel qu'expliqué en référence à la Figure 3, si la présence d'un épisode de tachyarythmie n'est pas suspectée (voir la flèche 202 sur la Figure 4a), le procédé 30 retourne à la première étape 100. A l'inverse, si la présence d'un épisode de tachyarythmie est suspectée (voir la flèche 204 sur la Figure 4a), le procédé du traitement des signaux 30 se poursuit à la troisième étape 300.

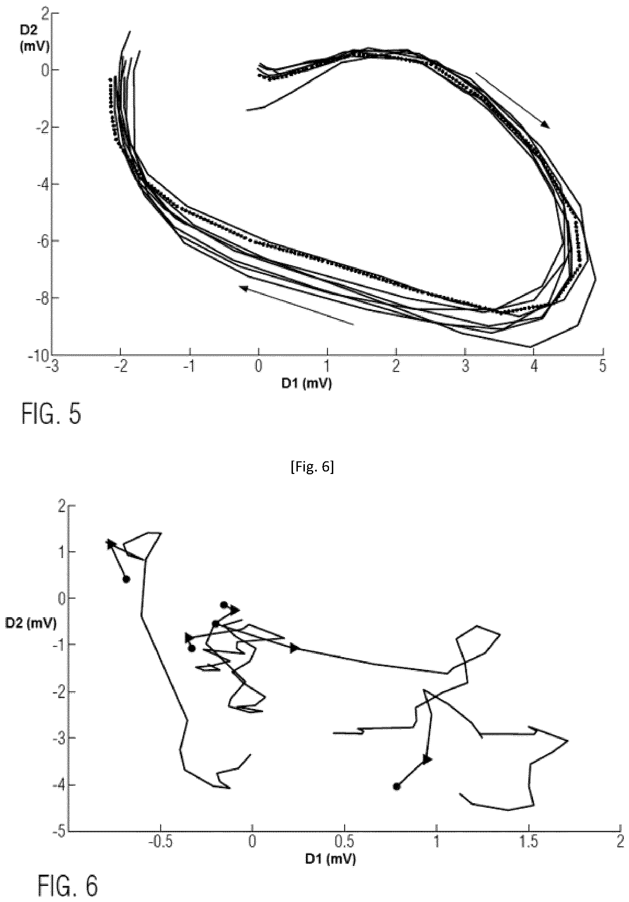

A la troisième étape 300, les battements successifs enregistrés simultanément par voie sous-cutanée via le premier dipôle D1 et le deuxième dipôle D2, dont une fraction des battements est comprise à l'intérieur de la fenêtre temporelle comprenant le complexe QRS et centrée sur l'onde R, sont représentés sous forme d'une courbe bidimensionnelle avec le premier dipôle D1 en abscisse et le deuxième dipôle D2 en ordonnée. Dans une variante, le deuxième dipôle D2 pourrait être en abscisse et le premier dipôle D1 en ordonnée. Une telle courbe bidimensionnelle est représentée à la Figure 5. Tel qu'illustré à la Figure 5, la courbe bidimensionnelle n'est pas une boucle fermée car elle ne correspond qu'à une partie de la boucle cardiaque complète, c'est-à-dire au complexe QRS isolé à l'intérieur de la fenêtre temporelle.

L'analyse bidimensionnelle des signaux recueillis, c'est-à-dire en deux dimensions, ne doit pas être entendue de manière en elle-même limitative, l'invention s'appliquant aussi bien à une analyse dans un espace multidimensionnel d'ordre supérieur (3D ou plus), par extrapolation des enseignements de la présente description à une situation où des signaux sont recueillis de manière sous-cutanée concurremment sur trois dipôles ou plus.

Comme montré par la Figure 4a, l'étape 400 du procédé 30, à laquelle est identifiée la présence ou non de bruit indésirable dans les signaux recueillis, comprend trois sous-étapes successives 401, 403 et 405.

A la première sous-étape 401 de l'étape 400, les coordonnées d'un vecteur tangent en chaque point de la courbe bidimensionnelle déterminée à l'étape 300, sont calculées.

A la deuxième sous-étape 403 de l'étape 400, l'algorithme du procédé 30 calcule le nombre de fois qu'au moins une des deux coordonnées de chaque vecteur tangent change de signe entre deux points successifs de la courbe bidimensionnelle.

La deuxième sous-étape 403 permet ainsi de caractériser la courbure de la courbe bidimensionnelle. En effet, dans le cas d'un cycle cardiaque non bruité, il existe entre les signaux recueillis sur les deux dipôles D1, D2 une relation qui fait que la courbe bidimensionnelle est représentée de façon essentiellement régulière. À l'inverse, dans le cas d'un cycle cardiaque bruité, c'est-à-dire comprenant des artefacts d'origine extracardiaque, la courbe bidimensionnelle peut être représentée de façon erratique, tel que représenté à la Figure 6. Une telle représentation erratique (voir Figure 6) est alors caractérisée, selon la présente invention, par le nombre de fois qu'au moins une des coordonnées du vecteur tangent change de signe entre chaque point successif de la courbe bidimensionnelle.

A la troisième sous-étape 405 de l'étape 400, l'algorithme du procédé 30 compare ce nombre de changement de signe à un nombre seuil prédéterminé indicatif de bruit indésirable. Ce nombre seuil prédéterminé indicatif de bruit indésirable peut être défini relativement à la totalité de la pluralité de points de ladite courbe bidimensionnelle. Dans une variante, ce nombre seuil prédéterminé indicatif de bruit indésirable peut être défini comme un seuil absolu pour un certain nombre de points consécutifs donnés.

Si la présence de bruit indésirable dans les signaux n'est pas détectée (voir la flèche 408 sur les Figures 4a-b), le procédé du traitement des signaux 30 se poursuit à la cinquième étape 500.

A l'inverse, si la présence de bruit indésirable est détectée dans les signaux (voir la flèche 402 sur la Figure 4a), la sous-étape 405 de l'étape 400 est suivie d'une étape 404 à laquelle la/les composante(s) de bruit indésirable dans les signaux recueillis détecté à l'étape 400 est/sont ignorée(s). Dans une variante, à la suite un certain nombre donné de cycles bruités détectés, l'algorithme enclenche un mode dit « mode bruit », dans lequel des paramètres, notamment le seuil de détection des ondes R, sont modifiés temporairement pendant un certain lapse de temps, par exemple pendant 30 secondes. Dans une autre variante, l'algorithme, en mode bruit, allonge les périodes réfractaires pendant un certain lapse de temps prédéfini.

L'étape 404 est suivie d'une étape 407 (voir la flèche 406 sur la Figure 4a) au cours de laquelle l'intervalle R-R est à nouveau calculé sur les signaux dont le bruit indésirable a été ignoré et n'a pas donc pas été pris en compte. L'étape 407 est alors suivie de l'étape 200, précédemment décrite, au cours de laquelle l'intervalle R-R des signaux, nettoyés du bruit indésirable, est comparé à un seuil indicatif de tachyarythmie.

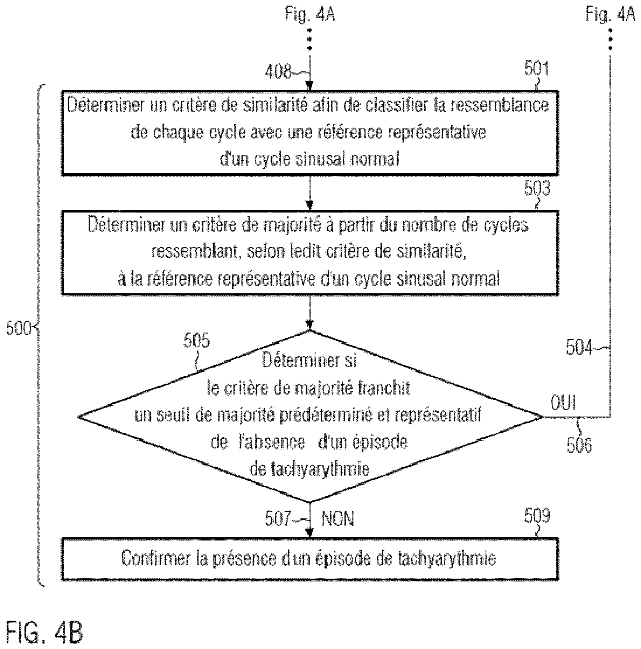

Comme montré à la Figure 4b, l'étape 500 du procédé 30, à laquelle la présence ou non de tachyarythmie est confirmée, comprend quatre sous-étapes successives 501, 503, 505, 509.

A la première sous-étape 501 de l'étape 500, où les signaux ne comprennent pas de bruit indésirable mais dont l'intervalle R-R est inférieur au seuil indicatif de tachyarythmie (voir étape 200 du procédé 30), une analyse de comparaison, en particulier de similarité, est démarrée. Ainsi, à la sous-étape 501, la courbe bidimensionnelle déterminée à l'étape 300 est comparée à une courbe bidimensionnelle de référence représentative d'un rythme sinusal normal, par exemple préalablement enregistrée dans la mémoire du contrôleur 28.

Dans une variante, l'étape 501 peut comprendre la détermination d'un critère de similarité en considérant, en plus d'une référence en cycle sinusale normale, une référence représentative d'une « onde P » et une référence représentative d'une « onde T ». Ainsi, l'étape 501 de cette variante permet d'identifier la surdétection potentielle P/T car il y aurait à la fois une « similarité à la référence T » ou une « similarité à la référence P ».

Afin de classifier la ressemblance de chaque cycle avec la référence représentative d'un rythme sinusal normal, un critère de similarité est déterminé à l'étape 501. Selon le critère de similarité, chaque cycle sera classifié soit en « ressemble au rythme sinusal normal » soit en « ne ressemble pas au rythme sinusal normal ».

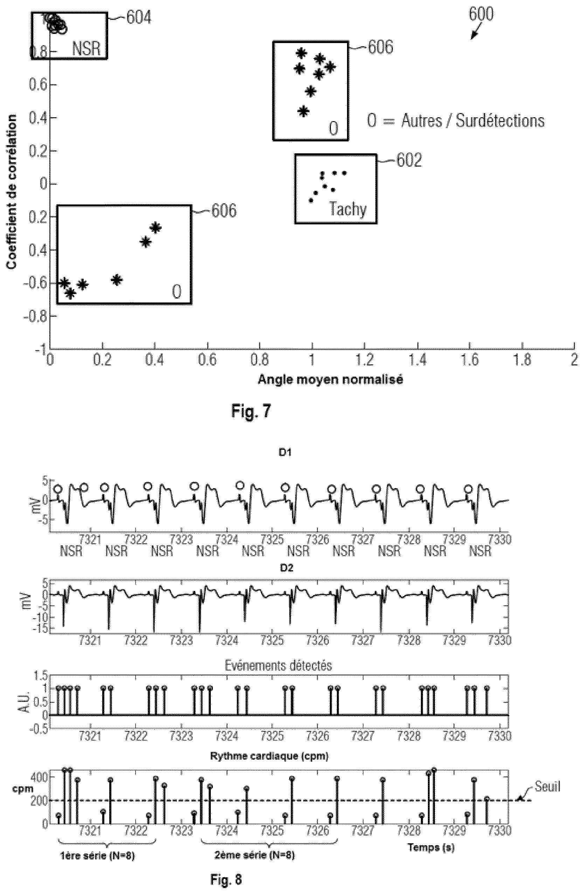

Pour déterminer le critère de similarité, les vecteurs tangents de chaque courbe en chaque point sont déterminés. Puis, un angle moyen entre les vecteurs tangents des deux courbes en chaque point est déterminé. Ensuite, un coefficient de corrélation entre les normes des vecteurs tangents des deux courbes en chaque point est déterminé. Enfin, l'angle moyen et le coefficient de corrélation sont représentés sur un graphique, tel qu'illustré à la Figure 7, à partir duquel on peut déterminer si les courbes sont similaires entre elles ou non. Le graphique de la Figure 7 représente la classification de chaque cycle en « similaire » à la référence sinusale ou « non similaire » à la référence sinusale. Dans le cas de « non similaire », il n'est pas possible, à ce stade du procédé 30, de savoir si le cycle est représentatif d'une surdétection ou d'une tachyarythmie. C'est pourquoi, à une sous-étape 503 de l'étape 500, un critère de majorité est déterminé.

Le critère de majorité peut être fonction, sur les N derniers battements non-bruités enregistrés, du nombre de cycles Ci similaires à ceux de la référence représentative d'un rythme sinusal normal. Dans une variante, le critère de similarité peut également être défini par un ratio Ci sur N (Ci/N) avec N le nombre de derniers battements non-bruités enregistrés. La similarité des cycles avec ceux de la référence est ainsi déterminée au moyen du critère de similarité à l'étape 501.

A une sous-étape 505 de l'étape 500, ce critère de majorité, c'est-à-dire la valeur de Ci ou le ratio Ci/N, est comparé à un seuil de majorité prédéterminé et représentatif de l'absence d'un épisode de tachyarythmie Créf.

Si une majorité de cycles similaires à la référence sinusale (par exemple 8 Ci sur les 12 derniers cycles) est détectée, alors la présence tachyarythmie n'est pas confirmée. Le procédé 30 est alors réinitialisé de sorte que l'étape 505 est suivie de la première étape 100 de l'algorithme (voir la flèche 506 sur la Figure 4b), c'est-à-dire de l'acquisition de nouveaux signaux électriques.

Au contraire, s'il n'y a pas une majorité de cycles similaires à la référence sinusale (par exemple 2 Ci sur les 12 derniers cycles), alors la tachyarythmie est confirmée à l'étape 509.

Ainsi, selon que le critère de majorité franchisse un seuil prédéterminé Créf, l'algorithme conclut à l'absence ou la présence d'un épisode de tachyarythmie.

La Figure 8 illustre un exemple où la première série de 8 battements (N=8) comprend 3 cycles qui ressemblent au cycle représentatif d'un rythme sinusal normal (Ci=3). Les 5 cycles qui ne ressemblent pas au cycle représentatif d'un rythme sinusal normal sont indiqués par des symboles « o ». Ainsi, dans l'exemple illustré à la Figure 7, le critère de majorité de la première série est équivalent à un ratio 3/8.

Dans la deuxième série de 8 battements illustrée à la Figure 8, le critère de majorité est équivalent à un ratio 4/8.

Dans le cas où Créf est prédéterminé comme une valeur supérieure ou égale à 3/8, alors l'algorithme ne confirme pas le présence d'un épisode de tachyarythmie suite à l'analyse de la première série de battement et la deuxième série de battement de la Figure 8.

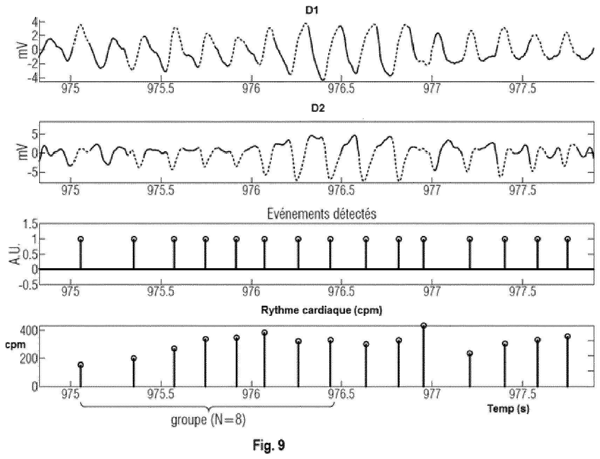

La Figure 9 illustre un exemple où aucun cycle ne ressemble au cycle représentatif d'un rythme sinusal normal. Ainsi, dans l'exemple de la Figure 8, N=8 et Ci=0. Le critère de majorité de l'exemple de la Figure 9 est donc nul, et ainsi nécessairement inférieur au seuil Créf. Dans un tel cas, la présence d'un épisode de tachyarythmie est alors confirmée à l'étape 509 et vérifiée sur une pluralité de fenêtres glissantes.

La courbe bidimensionnelle de référence représentative d'un rythme sinusal normal est déterminée en faisant la moyenne ou/et la médiane des au moins deux derniers cycles de rythme sinusal normal du patient.

Dans un mode de réalisation où le dispositif médical implantable sous cutanée 10 comprend un accéléromètre et/ou gyroscope, le contrôleur 28 du dispositif 10 pourrait en outre être configuré pour déterminer le critère de similarité en comparant ladite courbe bidimensionnelle à une courbe bidimensionnelle de référence dans ladite position déterminée et représentative d'un rythme sinusal normal. Ainsi, en permettant la détection de la position du patient, l'interprétation des signaux électriques recueillis peut être davantage affinée, et donc améliorée. De plus, les courbes bidimensionnelles de référence déterminées en rythme sinusal normal pour chaque position peuvent être comparées entre elles, afin de ne garder en mémoire que celles qui sont suffisamment différentes d'une position à l'autre. Cela permet notamment d'économiser de la mémoire de stockage. En outre, les courbes de référence pour chaque position peuvent être actualisées, par exemple de manière journalière ou hebdomadaire.

A la suite de l'étape 509, le contrôleur 28 du dispositif 10 peut être configuré pour déclencher une opération de défibrillation au moyen de l'électrode de défibrillation 26 de la sonde implantable sous cutanée 14.

Le contrôler 28 du dispositif 10 est configuré pour réaliser chacune des étapes et sous-étapes du procédé 30.

## Revendications

1. Un dispositif médical actif implantable sous cutané, en particulier un défibrillateur cardiaque sous cutané, comprenant :
un boîtier (12),
une sonde implantable sous cutanée (14) connectée au boîtier (12),
la sonde implantable sous cutanée (14) comprenant une pluralité d'électrodes de détection (20, 22, 24) formant au moins deux dipôles (D1, D2) à partir desquels au moins deux signaux électriques sont concurremment recueillis ;
le premier dipôle (D1) ayant une longueur (L1) inférieure à celle (L2) du deuxième dipôle (D2);
le dispositif médical actif implantable sous cutané comprenant en outre un contrôleur (28) configuré pour combiner les signaux recueillis via le premier dipôle (D1) et les signaux recueillis via le deuxième dipôle (D2), et
à partir de la représentation des signaux recueillis via le deuxième dipôle (D2) en fonction des signaux recueillis via le premier dipôle (D1), de déterminer une courbe bidimensionnelle paramétrée en fonction du temps représentative de l'activité cardiaque d'un patient,
le contrôleur étant configuré pour déterminer la présence ou non d'une tachyarythmie en déterminant un critère de similarité à partir des signaux électriques recueillis concurremment via le premier dipôle (D1) et via le deuxième dipôle (D2) pendant une série définie de cycles cardiaques , telle que la détection d'un pic de dépolarisation, correspondant à la détection d'une onde R, est réalisée via le premier dipôle (D1), le critère de similarité étant défini par une comparaison entre ladite courbe bidimensionnelle et une courbe bidimensionnelle de référence représentative d'un rythme sinusal normal.

2. Le dispositif médical actif implantable sous cutané selon la revendication 1, dont les signaux recueillis via le premier dipôle (D1) et le deuxième dipôle (D2) de la sonde implantable sous cutanée (14) sont considérées sur une fenêtre temporelle comprenant un complexe QRS et centrée sur la détection de l'onde R,
la détection de l'onde R étant uniquement réalisée via le premier dipôle (D1).

3. Le dispositif médical actif implantable sous cutané selon la revendication 1 ou 2, dont le contrôleur (28) est en outre configuré pour identifier la présence ou non de bruit indésirable dans les signaux recueillis en fonction du changement de signe d'au moins une des coordonnées d'un vecteur tangent entre chaque point successif d'une pluralité de points de ladite courbe bidimensionnelle,
le contrôleur (28) étant configuré pour identifier la présence de bruit indésirable en déterminant le nombre de fois qu'au moins une des coordonnées du vecteur tangent change de signe entre chaque point successif de la pluralité de points de ladite courbe bidimensionnelle et en comparant ce nombre à un nombre seuil prédéterminé indicateur de bruit indésirable.

4. Le dispositif médical actif implantable sous cutané selon l'une des revendications précédentes, dont le contrôleur (28) est en outre configuré pour déterminer la présence ou non d'une tachyarythmie en déterminant un critère de majorité qui est déterminé à partir du critère de similarité en calculant le nombre de cycles semblables entre les signaux recueillis et des signaux de référence représentatifs d'un cycle sinusal normal.

5. Le dispositif médical actif implantable sous cutané selon l'une des revendications 1 à 4, dont la sonde implantable sous cutanée (14) comprend en outre une électrode de défibrillation (26).

6. Le dispositif médical actif implantable sous cutané selon la revendication 5, dont le contrôleur (28) est configuré pour déclencher une opération de défibrillation au moyen de l'électrode de défibrillation (26) lorsque le critère de similarité indique la présence d'une tachyarythmie à traiter.

7. Le dispositif médical actif implantable sous cutané selon l'une des revendications 1 à 6, comprenant en outre un accéléromètre ou/et un gyroscope de manière à ce que le contrôleur (28) est configuré pour déterminer une position du patient au moyen de l'accéléromètre ou/et du gyroscope ;
le contrôleur étant en outre configuré pour déterminer le critère de similarité en comparant ladite courbe bidimensionnelle à une courbe bidimensionnelle de référence dans ladite position déterminée et représentative d'un rythme sinusal normal.

## Patentansprüche

1. Aktives subkutan implantierbares medizinisches Gerät, insbesondere ein subkutaner kardialer Defibrillator, umfassend:
ein Gehäuse (12),
eine subkutan implantierbare Sonde (14), die mit dem Gehäuse (12) verbunden ist,
wobei die subkutan implantierbare Sonde (14) eine Vielzahl von Abtastelektroden (20, 22, 24) aufweist, die mindestens zwei Dipole (D1, D2) bilden, von denen mindestens zwei elektrische Signale gleichzeitig gesammelt werden;
wobei der erste Dipol (D1) eine kürzere Länge (L1) als die (L2) des zweiten Dipols (D2) aufweist;
wobei das aktive subkutan implantierbare medizinische Gerät ferner eine Steuerung (28) umfasst, die konfiguriert ist, um die über den ersten Dipol (D1) gesammelten Signale und die über den zweiten Dipol (D2) gesammelten Signale zu kombinieren, und
anhand der Darstellung der über den zweiten Dipol (D2) gesammelten Signale in Abhängigkeit von den über den ersten Dipol (D1) gesammelten Signalen eine zweidimensionale Kurve zu bestimmen, die als Funktion der Zeit parametrisiert und für die Herzaktivität eines Patienten repräsentativ ist,
wobei die Steuerung konfiguriert ist, um das Vorhandensein oder Fehlen einer Tachyarrhythmie zu bestimmen, indem ein Ähnlichkeitskriterium aus elektrischen Signalen bestimmt wird, die gleichzeitig über den ersten Dipol (D1) und über den zweiten Dipol (D2) während einer definierten Serie von Herzzyklen gesammelt wurden, so wie z. B. die Erfassung eines Depolarisationspeaks, die der Erfassung einer R-Welle entspricht, über den ersten Dipol (D1) realisiert wird,
wobei das Ähnlichkeitskriterium durch einen Vergleich zwischen der zweidimensionalen Kurve und einer für einen normalen Sinusrhythmus repräsentativen zweidimensionalen Referenzkurve definiert ist.

2. Aktives subkutan implantierbares medizinisches Gerät nach Anspruch 1, wobei die über den ersten Dipol (D1) und den zweiten Dipol (D2) der subkutan implantierbaren Sonde (14) gesammelten Signale über ein Zeitfenster betrachtet werden, das einen QRS-Komplex umfasst und auf die Erfassung der R-Welle fokussiert ist, wobei die Erfassung der R-Welle nur über den ersten Dipol (D1) erfolgt.

3. Aktives subkutan implantierbares medizinisches Gerät nach Anspruch 1 oder 2, wobei die Steuerung (28) ferner konfiguriert ist, um das Vorhandensein oder Fehlen von unerwünschtem Rauschen in den gesammelten Signalen basierend auf der Änderung des Vorzeichens mindestens einer der Koordinaten eines Tangentenvektors zwischen jedem aufeinanderfolgenden Punkt einer Vielzahl von Punkten auf der zweidimensionalen Kurve zu identifizieren,
wobei die Steuerung (28) konfiguriert ist, um das Vorhandensein von unerwünschtem Rauschen zu identifizieren, indem sie bestimmt, wie oft mindestens eine der Koordinaten des Tangentenvektors das Vorzeichen zwischen jedem aufeinanderfolgenden Punkt der Vielzahl von Punkten auf der zweidimensionalen Kurve ändert und diese Zahl mit einem vorbestimmten Schwellenwert vergleicht, der unerwünschtes Rauschen anzeigt.

4. Aktives subkutan implantierbares medizinisches Gerät nach einem der vorhergehenden Ansprüche, wobei die Steuerung (28) ferner konfiguriert ist, um das Vorhandensein oder Fehlen einer Tachyarrhythmie zu bestimmen, indem ein Mehrheitskriterium bestimmt wird, das mittels des Ähnlichkeitskriteriums bestimmt wird, indem die Anzahl ähnlicher Zyklen zwischen den gesammelten Signalen und für einen normalen Sinuszyklus repräsentativen Referenzsignalen berechnet wird.

5. Aktives subkutan implantierbares medizinisches Gerät nach einem der Ansprüche 1 bis 4, wobei die subkutan implantierbare Sonde (14) zusätzlich eine Defibrillationselektrode (26) umfasst.

6. Aktives subkutane implantierbares medizinisches Gerät nach Anspruch 5, wobei die Steuerung (28) so konfiguriert ist, dass sie mittels der Defibrillationselektrode (26) eine Defibrillationsoperation auslöst, wenn das Ähnlichkeitskriterium das Vorhandensein einer zu behandelnden Tachyarrhythmie anzeigt.

7. Aktives subkutan implantierbares medizinisches Gerät nach einem der Ansprüche 1 bis 6, ferner umfassend einen Beschleunigungssensor und/oder ein Gyroskop in der Weise, dass die Steuerung (28) konfiguriert ist, um eine Position des Patienten mittels des Beschleunigungssensors und/oder des Gyroskops zu bestimmen;
wobei die Steuerung ferner konfiguriert ist, um das Ähnlichkeitskriterium zu bestimmen, indem die zweidimensionale Kurve mit einer für einen normalen Sinusrhythmus repräsentativen zweidimensionalen Referenzkurve in der bestimmten Position verglichen wird.

## Claims

1. A subcutaneous implantable active medical device, in particular a subcutaneous cardiac defibrillator, comprising:
a housing (12);
a subcutaneous implantable lead (14) connected to the housing (12);
the subcutaneous implantable lead (14) comprising a plurality of sensing electrodes (20, 22, 24) forming at least two dipoles (D1, D2) from which at least two electrical signals are collected concurrently;
the first dipole (D1) having a length (L1) less than a length (L2) of the second dipole (D2);
the subcutaneous implantable active medical device further comprising a controller (28) configured to combine the signals collected via the first dipole (D1) and the signals collected via the second dipole (D2), and
on the basis of the representation of the signals collected via the second dipole (D2) as a function of the signals collected via the first dipole (D1), to determine a two-dimensional curve that is parameterized as a function of time and that is representative of the cardiac activity of a patient,
the controller being configured to determine whether or not tachyarrhythmia is present by determining a criterion of similarity based on the electrical signals collected concurrently via the first dipole (D1) and via the second dipole (D2) during a defined series of cardiac cycles that is such that detection of a depolarization peak,
corresponding to detection of an R wave, is performed via the first dipole (D1),
the criterion of similarity being defined by a comparison between said two-dimensional curve and a reference two-dimensional curve representative of a normal sinus rhythm.

2. The subcutaneous implantable active medical device of claim 1, wherein the signals collected via the first dipole (D1) and via the second dipole (D2) of the subcutaneous implantable lead (14) are considered over a time window comprising a QRS complex and centered on the detection of the R wave,
the detection of the R wave being performed via the first dipole (D1) only.

3. The subcutaneous implantable active medical device of any of claims 1 and 2, wherein the controller (28) is further configured to identify a presence or absence of undesirable noise in the collected signals as a function of the change of sign of at least one of the coordinates of a tangent vector between each successive point of a plurality of points of said two-dimensional curve,
the controller (28) being configured to identify the presence of undesirable noise by determining the number of times at least one of the coordinates of the tangent vector changes sign between each successive point of the plurality of points of said two-dimensional curve and by comparing said number with a predetermined threshold number indicative of undesirable noise.

4. The subcutaneous implantable active medical device of any of the preceding claims, wherein the controller (28) is further configured to determine the presence or absence of tachyarrhythmia by determining a criterion of majority that is determined on the basis of the criterion of similarity by computing the number of cycles that are similar between the collected signals and reference signals that are representative of a normal sinus cycle.

5. The subcutaneous implantable active medical device of any of claims 1 to 4, wherein the subcutaneous implantable lead (14) further comprises a defibrillation electrode (26).

6. The subcutaneous implantable active medical device of claim 5, wherein the controller (28) is configured to trigger a defibrillation operation by means of the defibrillation electrode (26) when the criterion of similarity indicates the presence of tachyarrhythmia to be treated.

7. The subcutaneous implantable active medical device of any of claims 1 to 6, further comprising an accelerometer and/or a gyroscope in such a manner that the controller (28) is configured to determine a position of the patient by means of the accelerometer and/or of the gyroscope,
the controller being further configured to determine the criterion of similarity by comparing said two-dimensional curve with a reference two-dimensional curve in said determined position, which reference two-dimensional curve is representative of a normal sinus rhythm.
